# EUROPEAN PATENT APPLICATION

(11) **EP 1 887 287 A2**
(43) Date of publication of application: **13.02.2008**
(21) Application number: 07013618.9
(22) Date of filing: 11.07.2007
(51) Int. Cl.: F24F 3/16

(54) **Air conditioner having air filtering unit**

(30) Priority: 01.08.2006 JP 2006209443
(71) Applicant: Sanyo Electric Co., Ltd., Moriguchi-shi, Osaka-fu (JP)
(72) Inventor: Nagae, Koji, Gunma 370-0514 (JP)
(74) Representative: Glawe, Delfs, Moll

(57) **Abstract**

An air conditioner including a refrigerant circuit including a compressor, an outdoor heat exchanger and an indoor heat exchanger which are successively connected to one another, a housing for accommodating the indoor heat exchanger, an air suction port for sucking air, an air blow-out port for blowing out air to a room, an air blowing fan for making air flow from the air suction port to the air blow-out port to form an air flow passage, and an air filtering unit that is disposed in the air flow passage extending from the air suction port to the air blow-out port and bringing air heat-exchanged by the indoor heat exchanger into contact with liquid having a filtering effect.

## Description

### BACKGROUND OF THE INVENTION

### 1. Field of the Invention

The present invention relates to an air conditioner that is equipped with a refrigerant circuit including a compressor, a four-way valve, an outdoor heat-exchanger and an indoor heat-exchanger which are successively connected to one another, and can remove microorganisms floated in the air (bacteria, virus, fungus, etc. (hereinafter referred to as "virus, etc.")) with liquid having an air filtering effect. Here, the air filtering effect is broadly defined as containing various effects of removal, inactivation, sterilization, etc. of virus, etc.

### 2. Description of the Related Art

There is known a method of bringing air into contact with liquid having an air filtering effect such as alkaline electrolytic water or the like to decompose, remove, inactivate, sterilize or the like (hereinafter referred to as "filter") harmful materials contained in the air, thereby purifying (filtering) the air. Furthermore, there is known a method of subjected the filtered air to cooling/heating operation, dehumidification or the like, thereby supplying comfortable air to the room (for example, see JP-A-2003-250876).

When air conditioning operation such as cooling/heating operation or the like is carried out, comfort of temperature and humidity is importable. Even under the same indoor temperature, the feeling temperature is higher under a higher humidity atmosphere. Therefore, it is preferable to dehumidify the room under cooling operation in the summer season where the humidity is high, and also it is preferable to humidify the room together with heating operation in the winter season where the humidity is low.

However, when air is purified according to a wet system as in the case of a conventional method, for example, under cooling operation, the air conditioner cools humidified air and thus the temperature of blow-out air is increased because the sensible heat factor of the heat exchanger is constant. Therefore, the cooling load is increased in order to keep comfort of the air atmosphere of the room. Furthermore, during heating operation, the room air is humidified by the purification of the air, however, the humidifying effect of air to be supplied to the room is lowered because the humidified air is further humidified.

### SUMMARY OF THE INVENTION

Therefore, the present invention has been implemented in view of the foregoing problems, and has an object to provide an air conditioner that can filter/purify air according to a wet system and keep the air atmosphere of a room comfortable without increasing an air conditioning load.

In order to attain the above obj ect, according to the present invention, there is provided an air conditioner comprising: a refrigerant circuit including a compressor, an outdoor heat exchanger and an indoor heat exchanger which are successively connected to one another; a housing for accommodating the indoor heat exchanger; an air suctionport for sucking air; an air blow-out port for blowing out air to a room; an air blowing fan for making air flow from the air suction port to the air blow-out port to form an air flowpassage; and an air filtering unit that is disposed in the air flow passage extending from the air suction port to the air blow-out port and bringing air heat-exchanged by the indoor heat exchanger into contact with liquid having a filtering effect.

According to the air conditioner, the air flow passage is formed so as to extend from the air suction port to the air blow-out port by the air blowing fan, and the air filtering unit is disposed at the downstream side of the indoor heat exchanger with respect to the air flow in the air flow passage. Therefore, when cooling operation is carried out by switching the four-way valve, air which is cooled by the heat exchanger and thus has relatively high humidity is supplied to the air filtering unit, and also when heating operation is carried out by switching the four-way valve, air which is heated (warmed) by the heat exchanger and thus has relatively low humidity is supplied to the air filtering unit. Accordingly, when air is brought into contact with liquid having an air filtering effect in the air filtering unit, during cooling operation, air which has been already kept to a relatively high humidity state is supplied to the air filtering unit and thus the relative humidity of the air after an air filtering operation in the air filtering unit can be suppressed from increasing. During heating operation, air which has been already kept to a relatively low humidity state is supplied to the air filtering unit and thus the relative humidify of the air after the air filtering operation in the air filtering unit can be increased. Accordingly the air can be filtered/purified according to the wet system, and also the humidification amount of air under air conditioning operation can be automatically controlled without increasing the air conditioning load, so that the air atmosphere of the room can be kept comfortable.

In the above air conditioner, the air filtering unit may be equipped with a gas-liquid contact member for bringing the air heat-exchanged by the indoor heat exchanger into contact with the liquid, and a liquid supply unit for supplying the liquid to the gas-liquid contact member.

Furthermore, in the above air conditioner, the liquid preferably comprise electrolytic water containing active oxygen species.

In this case, it is preferable that the active oxygen species contain at least one material selecting from the group consisting of hypochlorous acid, ozone and hydrogen peroxide.

In the above air conditioner, the liquid supply unit may comprise an electrolytic unit for electrolyzing tap water to generate the electrolytic water.

Furthermore, in the above air conditioner, the electrolytic unit may include at least one pair of electrodes, the water being electrolyzed by supplying current to the electrodes.

In the above air conditioner, the liquid supply unit may further comprise an electrical conductivity meter for detecting the electrical conductivity of the tap water to be supplied to the electrolytic unit, and an flow rate adjusting valve for adjusting a flow rate of the tap water to be supplied to the electrolytic unit in accordance with the electrical conductivity detected by the electrical conductivitymeter and a predetermined target concentration of the active oxygen species.

In the above air conditioner, it is preferable that the compressor, and the outdoor heat exchanger is accommodated in an outdoor unit, and the housing, the indoor heat exchanger, the air blowing fan and the air filtering unit are accommodated in an indoor unit.

In the above air conditioner, it is preferable that the air filtering unit is disposed at the downstream side of the indoor heat exchanger with respect to an air flow direction in the air flow passage.

According to the present invention, the air filtering/purification can be performed according to the wet system, the humidification amount of air under air conditioning operation can be automatically controlled without increasing the air conditioning load, and the air atmosphere of the room can be kept comfortable.

### BRIEF DESCRIPTION OF THE DRAWINGS

Fig. 1 is a diagram showing the construction of an air conditioner according to an embodiment of the present invention;
Fig. 2 is diagram showing the construction of an indoor unit according to the embodiment;
Fig. 3 is a diagram showing the construction of an air filtering unit according to the embodiment;
Fig. 4 is a diagram showing the construction of an electrolytic unit according to the embodiment; and
Figs. 5A to 5D are diagrams showing the effect of an air conditioning operation and an air filtering operation.

### DETAILED DESCRIPTION OF THE PREFERRED EMBODIMENT

A preferred embodiment according to the present invention will be described hereunder.

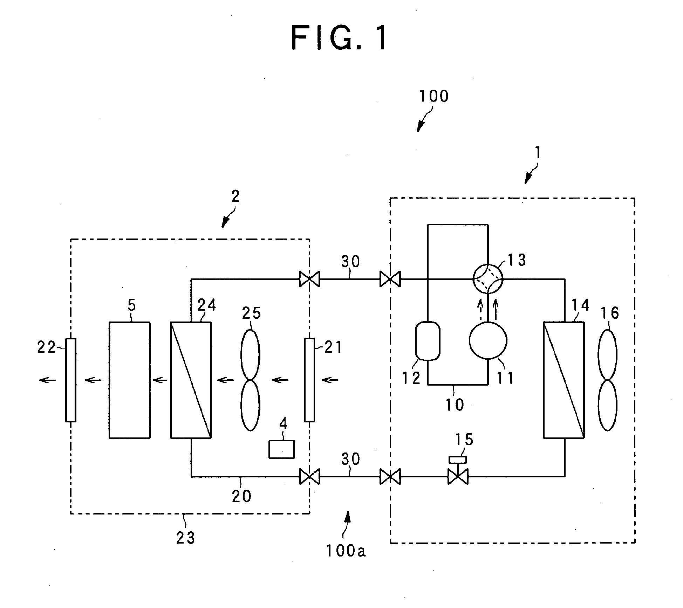

Fig. 1 shows the construction of an air conditioner 100 according to an embodiment of the present invention.

The air conditioner 100 of this embodiment is a separation type heat pump type air conditioner 100 having an outdoor unit 1 and an indoor unit 2. An outdoor refrigerant pipe 10 of the outdoor unit 1 and an indoor refrigerant pipe 20 of the indoor unit 2 are connected to each other through a link pipe 30, and a controller 4 controls the operation of each of the outdoor unit 1 and the indoor unit 2.

The outdoor unit 1 is disposed outdoors. As shown in Fig. 1, a compressor 11 is disposed in the outdoor refrigerant pipe 10, an accumulator 12 is connected to the suction side of the compressor 11, and a four-way valve 13, an outdoor heat exchanger 14 and an electrically-driven expansion valve 15 are successively connected to the discharge side of the compressor 11 in this order. Furthermore, an outdoor fan 16 for blowing air to the outdoor heat exchanger 14 is disposed in the outdoor unit 1.

The indoor unit 12 is disposed indoors. As shown in Fig. 1, a housing 23 having an air suction port 21 and air blow-out port 22 contains therein an indoor heat exchanger 24, an air blowing fan 25 for making air flow from the air suction port 21 to the air blow-out port 22 to form an air flow passage in the housing 23, and an air filtering unit 5 that is disposed in the air flow passage and bringing air heat-exchanged in the indoor heat exchanger 24 into contact with liquid having an air filtering effect (hereinafter referred to as "air filtering eater") to perform air filtering (i.e., removal, inactivation, sterilization, sterile filtration or the like of virus, etc. contained in the air).

The air conditioner 100 is designed so that the flow direction of refrigerant flowing in a refrigerant circuit 100a is switched by switching the four-way valve 13 to thereby switch cooling operation and heating operation to each other. Under cooling operation, refrigerant flows in a direction indicated by a solid-line arrow in Fig. 1, and under heating operation, refrigerant flows in a direction indicated by a chain-line arrow in Fig. 1.

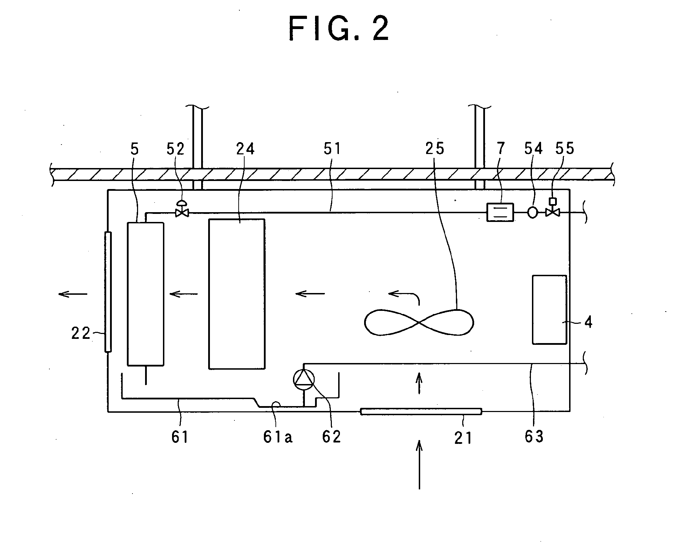

Next, the construction of the indoor unit 2 will be described with reference to Fig. 2.

The indoor unit 2 of this embodiment is designed as a ceiling-suspension type. It is hooked to suspension bolts suspended from the ceiling and fixedly set up at the lower surface of the indoor ceiling plate.

In Fig. 2, the air suction port 21 is formed at the bottom surface of the housing 23 of the indoor unit 2, and the air blow-out port 22 is formed at the front side of the housing 23 of the indoor unit 2. The air blowing fan 25 for making air flow from the air suction port 21 through the housing 23 to the air blow-out port 22 (i.e., along the air flow passage) is disposed at the upper side of the air suction port 21 in the housing 23, and the indoor heat exchanger 24 and the air filtering unit 5 are successively disposed in this order along the air flow passage from the air suction port 21 to the air blow-out port 22. The indoor heat exchanger 24 is a fin tube type heat exchanger, and it is connected to the indoor refrigerant pipe 20 as shown in Fig. 1. The refrigerant circuit 100a is not illustrated in Fig. 2.

A drain pan 61 of foamed polystyrene is disposed at the lower side of the indoor heat exchanger 24 and the air filtering unit 5 in the housing 23. A drain pump 62 is disposed in a drain pool 61 of the drain pan 61, and a drain hose 63 for discharging drain water to the outside of the indoor unit 2 is connected to the drain port of the drain pump 62.

The air filtering unit 5 is used to bring filtering water into contact with air which has been heat-exchanged by the indoor heat exchanger 24, thereby filtering the air which has been subjected to air conditioning operation. A filtering water supply pipe 51 (liquid supply unit) is connected to the air filtering unit 5, and filtering water is supplied from the filtering water supply pipe 51 to the air filtering unit 5. In the filtering water supply pipe 51 are successively disposed a flow amount adjusting valve 52 for adjusting the flow amount of filtering water supplied to the air filtering unit 5, an electrolytic unit 7 for generating from tap water or the like electrolytic water containing active oxygen species as filtering water, an electric conductivity meter for detecting the electric conductivity of tap water or the like, and an opening/closing valve 55 for selectively supplying tap water or the like to the electrolytic unit 7.

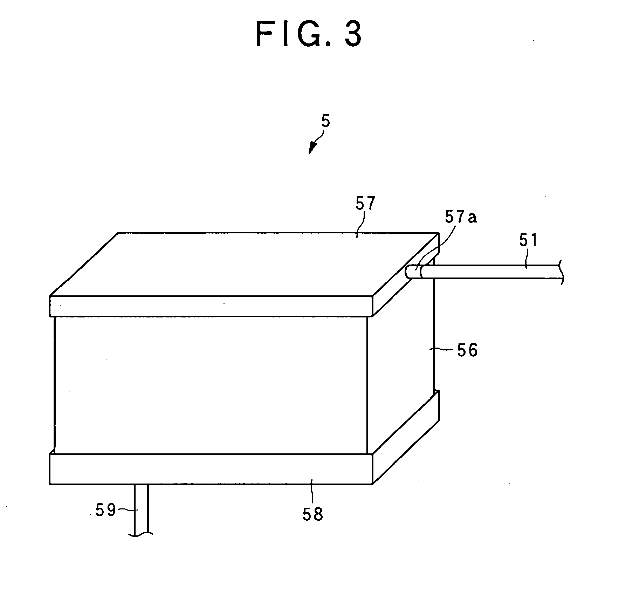

As shown in Fig. 3, the air filtering unit 5 is equipped with a gas-liquid contact member 56 having high water retentivity, a water dispersing tray 57 disposed at the upper side of the gas-liquid contact member 56, and a water receiving tray 58 disposed at the lower side of the gas-liquid contact member 56. The gas-liquid contact member 56 may be constructed by non-woven cloth formed of acrylic fiber, polyester fiber or the like. Furthermore, a raw material having little reactivity to the electrolytic water is preferably used as the raw material of the gas-liquid contact member 56, and polyolefin based resin (polyethylene resin, polypropylene resin or the like), vinyl chloride resin, fluorinated resin (PTFE, PFA, ETFE or the like), cellulose based material, ceramics based material or the like may be used for the gas-liquid contact member 56.

In this embodiment, the gas-liquid contact member 56 is subjected to a water affinity treatment, so that the gas-liquid contact member 56 has high affinity to electrolytic water. Accordingly, the water retentivity of the gas-liquid contact member 56 to the electrolytic water (wettability) is kept, and the contact between the filtering water and the introduced air can be kept for a long time.

The water dispersing tray 57 has a connection port 57a formed in the side surface thereof, and the filtering water supply pipe 51 is connected to the connection port 57a. Many holes (not shown) through which filtering water supplied through the filtering water supply pipe 51 is dropped and dispersed to the gas-liquid contact member 56 are formed in the bottom surface of the water dispersing tray 57.

The water receiving tray 58 holds the gas-liquid contact member 56 from the lower side, and it can stock filtering water passing through the gas-liquid contact member 56. A drain pipe 59 for guiding filtering water to the drain pan 61 (Fig. 2) is connected to the bottom surface of the water receiving tray 58.

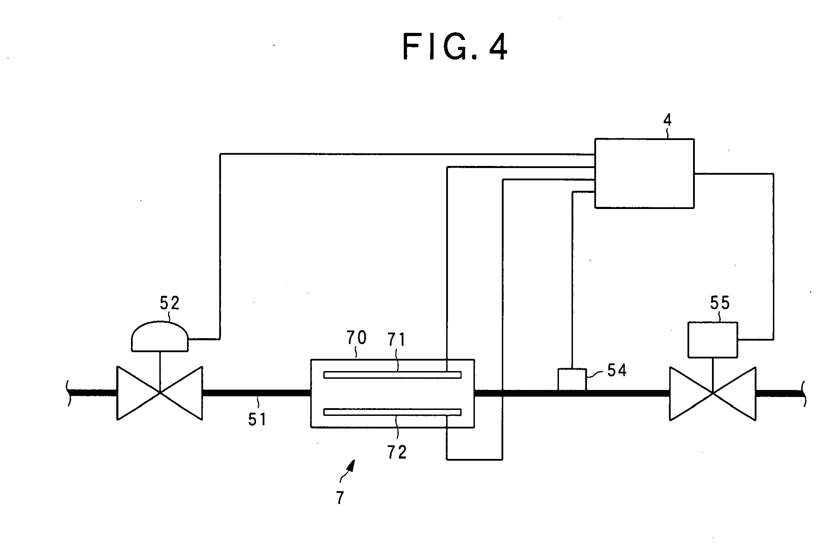

As shown in Fig. 4, the electrolytic water unit 7 has an electrolytic water bath 70 having a larger diameter than the filtering water supply pipe 51, and at least one pair of electrodes 71, 72 disposed in the electrolytic water bath 70. When current is supplied to the electrodes 71 and 72, the electrodes 71, 72 electrolyze tap water or the like which flows into the electrolytic tank 70 to generate electrolytic water containing active oxygen species.

Here, the active oxygen species means oxygen molecules having higher oxidizing activity than normal oxygen and also related substance thereof, and contain not only so-called narrowly-defined active oxygen such as superoxide anion, singlet oxygen, hydroxyl radical and hydrogen peroxide, but also so-called broadly-defined active oxygen such as ozone, hypochlorous acid, hypohalous acid, etc.

The electrodes 71, 72 may be constructed by two electrode plates each of which comprises a base of Ti (titan) and a coated layer of Ir (iridium), Pt (platinum).

When current is supplied to tap water by the electrodes 71, 72, the following reaction occurs at the cathode:

4H⁺ + 4e⁻ + (4OH⁻) → 2H₂ + (4OH⁻)

Furthermore, the following reaction occurs at the anode:

2H₂O → 4H⁺ + O₂ + 4e⁻

At the same time, chlorine ions contained water (chlorine ions are added in tap water in advance) reacts as follows:

2Cl⁻ → Cl₂ + 2e⁻

Furthermore, Cl₂ thus generated reacts with water as follows:

Cl₂ + H₂O → HClO + HCl

In this construction, by supplying current to the electrodes 71, 72, HClO (hypochlorous acid) having strong sterilizingpower is generated. Therefore, air ispassed through the gas-liquid contact member 56 supplied with the filtering water containing this hypochlorous acid to inactivate virus, etc. floated in the air passing through the gas-liquid contact member 56, thereby filtering the air, and also breeding of various bacteria, fungus, etc. in the gas-liquid contact member 56 can be prevented. Furthermore, when odor components pass through the gas-liquid contact member 56, the odor components also react with hypochlorous acid in the filtering water, and they are ionized and dissolved in the filtering water, whereby the odor components can be removed from air and thus the air is deodorized.

When current having a predetermined current density (for example, 20mA/cm² or the like) is supplied to the electrodes 71, 72, electrolytic water containing active oxygen species (hypochlorous acid) having a predetermined concentration (for example, free residual chlorine concentration of 1mg/l or the like) can be generated.

The flow rate adjusting valve 52 is used to adjust the flow rate of the filtering water supplied to the air filtering unit 5 by adjusting the opening degree thereof. In this construction, by adjusting the opening degree of the flow rate adjusting valve 52, the concentration of hypochlorous acid in the filtering water can be changed to a predetermined value (1 to 20mg/1). Specifically, when the flow rate adjusting valve 52 is adjusted so as to close the opening degree thereof, the flow rate of tap water or the like which flows between the electrodes 71 and 72 of the electrolytic unit 7 is reduced. On the other hand, when the voltage applied between the electrodes 71, 72 is constant irrespective of the flow rate of the tap water or the like, the amount of current flowing to the tap water or the like per unit volume is increased. Therefore, the electrolysis reaction at each of the electrodes 71, 72 is promoted, and the concentration of generated hypochlorous acid can be increased. Conversely, when the opening degree of the flow rate adjusting valve 52 is adjusted to be opened, the flow rate of tap water or the like flowing between the electrodes 71, 72 of the electrolytic unit 7 is increased, and thus the concentration of generated hypochlorous acid can be reduced.

The electrical conductivity meter 54 detects the electrical conductivity of tap water or the like which is supplied to the electrolytic unit 7. The opening degree of the flow rate adjusting valve 52 is adjusted on the basis of the electrical conductivity detected by the electrical conductivity meter 54 and a preset target concentration of active oxygen species so that the concentration of hypochlorous acid contained in the electrolytic water is equal to the target concentration concerned.

Next, the operation of the air conditioner 100 according to this embodiment will be describe.

In the air conditioner 100, the cooling operation or heating operation is carried out by switching the four-way valve 13 provided to the outdoor unit 1 to the cooling side or heating side.

First, the operation of the cooling operation will be described. When the four-way value 13 is switched to the cooling side, the refrigerant flows in the refrigerant circuit 100a as indicated by the solid-line arrow of Fig. 1. As a result, the outdoor heat exchanger 14 functions as a condenser, and the indoor heat exchanger 24 functions as an evaporator. Indoor air which is sucked from the air suction port 21 by the air blowing fan 25 in the indoor unit 2 is cooled by the indoor heat exchanger 24, so that the indoor air having relative higher humidity is increased is supplied to the air filtering unit 5. The air supplied to the air filtering unit 5 is brought into contact with the filtering water in the gas-liquid contact member 56, whereby virus, etc. are inactivated by the filtering water (electrolytic water). The relative humidity of air supplied to the gas-liquid contact member 56 is high, and thus even when the air is brought into contact with the filtering water in the gas-liquid contact member 56, the degree at which the air concerned is humidified in the gas-liquid contact member 56 is low, and thus the relative humidity of the air is little varied. The thus-filtered air is blown out from the air blow-out port 22 by the air blowing fan 25.

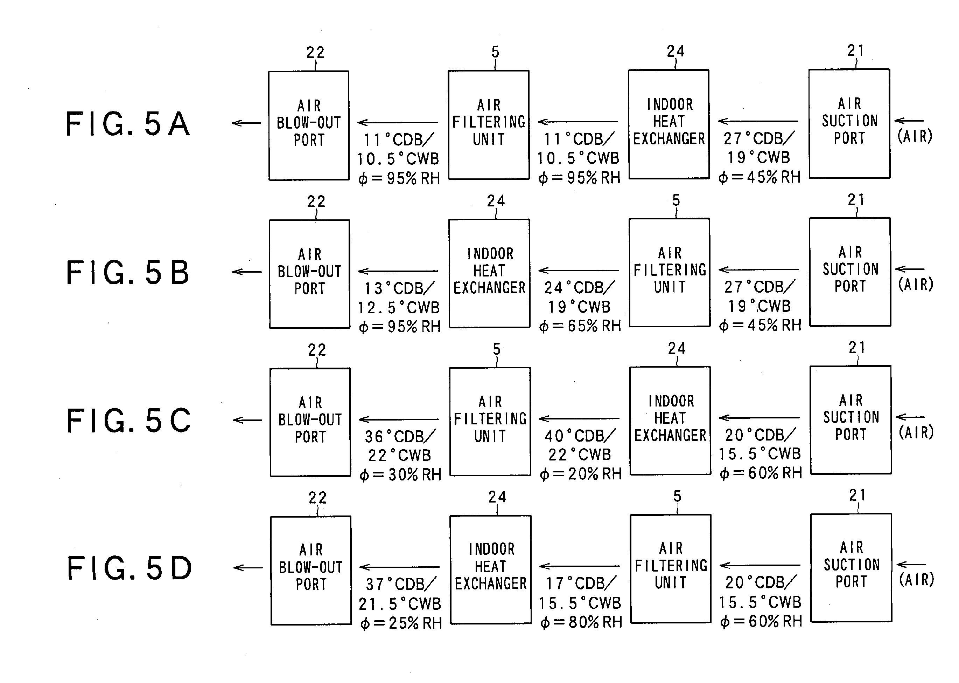

In the air conditioner 100 according to this embodiment, for example when air of 27°C DB (dry-bulb temperature), 19°C WB (wet-bulb temperature) and 45% RH (relative humidity) is supplied to the heat exchanger during cooling operation as shown in Fig. 5A, the air concerned is heat-exchanged by the heat exchanged and discharged as air of 11°C DB, 10.5°C WB and 95%RH. Even when the air after the heat exchange is supplied to the air filtering unit 5 and brought into contact with the filtering water, the temperature and humidity of the air are not varied, and the air-conditioned and filtered air of 11°C DB, 10.5°C WB and 95%RH is blown out from the air blow-out port 22 to the room. The arrows indicate the air flow in the housing 23 of the indoor unit 2.

On the other hand, when the arrangement of the indoor heat exchanger 24 and the air filtering unit 5 is inverted with respect to the air flow passage in the housing 23 as shown in Fig. 5B, that is, when air sucked from the air suction port 21 is first filtered in the air filtering unit 5, and then heat-exchanged by the indoor heat exchanger 24, the temperature and humidity of the air in the respective processes are as follows. First, when indoor air of 27°C DB, 19°C WB and 45%RH is supplied to the air filteringunit 5, it is brought into contact with the filtering water in the air filtering unit 5, and it becomes air of 24°C DB, 19°C WB and 65% RH. Thereafter, the air filtered in the air filtering unit 5 is heat-exchanged in the heat exchanger. At this time, when air which is more humid than the indoor air (the air whose relative humidity is increased from 45%RH to 65%RH) is heat-exchanged, the sensible heat factor (SHF) of the heat exchanger is constant, and thus the temperature and relative humidity of the air supplied from the heat exchanger are equal to 13°C DB, 12.5°C WB and 95%RH. Therefore, as compared with the air conditioner 100 of this embodiment, the temperature at the air blow-out side of the heat-exchanger is higher. Therefore, as compared with the air conditioner 100 of this embodiment, the cooling load must be increased in order to supply air having the same level of temperature to the room.

On the other hand, when the four-way valve 13 is switched to the heating side, the refrigerant flows in the refrigerant circuit 100a as indicated by the chain-line arrow of Fig. 1. As a result, the outdoor heat exchanger 14 functions as an evaporator, and the indoor heat exchanger 24 functions as a condenser. By the air blowing fan 25, the indoor air sucked from the air suction port 21 in the indoor unit 2 is heated (warmed) by the indoor heat exchanger 24, so that the indoor air which is lowered in relative humidity is supplied to the air filtering unit 5. The filtering water is supplied through the filtering water supply pipe 51 to the air filtering unit 5. The air supplied from the indoor heat exchanger 24 is subjected to air filtering in the gas-liquid contact member 56 in the same manner as described above, and also humidified by the gas-liquid contact member 56, so that the filtered and humidified air is blown out from the air suction port 22 by the air blowing fan 25.

In the air conditioner 100 of this embodiment, as shown in Fig. 5C, when air of 20°C DB (dry-bulb temperature), 15.5°C WB (wet-bulb temperature) and 45%RH (relative humidity) is supplied to the hat exchanger during heating operation, the air is heat-exchanged to air of 11°C DB, 10.5°C WB and 95%RH. Even when the air after the heat-exchange is supplied to the air filtering unit 5 and brought into contact with the filtering water, the temperature and humidity of the air are not varied, and the air-conditioned and filtered air of 11°C DB, 10.5°C WB and 95%RH is blown out from the air blow-out port 22.

On the other hand, when the arrangement of the indoor heat exchanger 24 and the air filtering unit 5 is inverted with respect to the air flow passage in the housing 23 as shown in Fig. 5D, the temperature and humidity of air are as follows. When indoor air of 20°C DB, 15.5°C WB and 60%RH is first supplied to the air filtering unit 5, the indoor air is brought into contact with the filtering water in the air filtering unit 5, so that the temperature of the air becomes 17°C DB and 15.5°C WB and the relative humidity of the air becomes 80%RH. Thereafter, the air filtered in the air f i ltering unit 5 is heat-exchanged in the heat exchanger. At this time, the air is humidified from 60%RH to 80%RH in the air filtering unit 5, however, the relative temperature of the air is reduced to 25%RH in the heat exchanger, and thus air of 37°C DB, 21. 5°C WB and 80%RH is supplied to the room. As described above, the temperature of the air blown out from the air blow-out port 22 side is the same level, however, the area of the gas-liquid contact portion of the gas-liquid contact member 56 must be increased in order to achieve the same level of the humidifying effect as the air conditioner 100 of this embodiment.

As described above, according to this embodiment, in the air conditioner 100 having the air filtering unit 5, a predetermined concentration of filtering water is supplied to the air filtering unit 5 and the heat-exchanged air is passed through the gas-liquid contact member 56, whereby the heat-exchanged indoor air is brought into contact with the filtering water to inactivate the virus, etc. In this construction, for example when influenza virus invades into indoor air, the active oxygen species function to break down and vanish (remove) the surface protein (spike) of the virus concerned which is indispensable for infection. When the surface protein of influenza virus is broken down, the influenza virus is not joined to a receptor which is necessary for infection of the virus concerned, so that infection can be prevented. As a result of a verification test which was made in cooperation with Sanitary Environment Research, it has been found that when air in which influenza virus invades is passed through the gas-liquid contact member 56 of this embodiment, 99% or more of the virus concerned can be removed.

Furthermore, according to this embodiment, indoor air sucked from the suction port 21 provided to the bottom surface of the housing 23 is brought into contact with electrolytic water dropped to the gas-liquid contact member 56 in the air filtering unit 5, and then blown out from the blow-out port 22 provided to the front side of the housing 23. Therefore, even when the suspension type air conditioner 100 is set up in a so-called large space such as a kindergarten, an elementary/junior high/high school, long-term care insurance facilities, a hospital or the like, air which has been air-conditioned and filtered (inactivated, sterilized or the like) can be blown out far away in the large space, so that filtering (sterile filtration or the like) of air can be efficiently performed in the large space.

As described above, when indoor air is filtered according to the wet system, according to the air conditioner 100 of this embodiment, the air flow passage is formed so as to extend from the air suction port 21 to the air blow-out port by the air blowing fan 25, and the air filtering unit 5 is disposed at the downstream side of the indoor heat exchanger 24 with respect to the air flow direction in the air flow passage. Therefore, when the air conditioner 100 is made to execute cooling operation by switching the four-way valve 13, air which is cooled in the heat exchanger to have higher relative humidity is supplied to the air filtering unit 5. During heating operation, air which is heated in the heat exchanger to have low relative humidity is supplied to the air filtering unit 5. Accordingly, even when the air is brought into contact with liquid having a filtering effect in the air filtering unit 5, during cooling operation, the relative humidity of the air canbe suppressed from increasing after the air filtering operation because air which has been already high in relative humidity is supplied to the air filtering unit 5, and also during heating operation, air having low relative humidity is supplied to the air filtering unit 5 and thus the relative humidity of the air can be increased after the air filtering operation. Accordingly, the air filtering/purification is carried out according to the wet system, and also the cooling operation and the heating operation are switched to each other by switching the four-way valve 13, whereby the humidification amount of the air under air conditioning operation can be automatically controlled without increasing the air conditioning load, and the air atmosphere of the room can be kept comfortable.

Furthermore, during cooling operation, air after the heat exchanger is filtered in the air filtering unit 5, and the indoor clean air can be supplied. Furthermore, the relative humidity of the air supplied to the air filtering unit 5 is high, and thus the consumption of the filtering water can be suppressed.

When air is humidified in the summer season where humidity is high, a user feels the sensible temperature high and also the discomfort index is high. Therefore, it has been hitherto general that the air filtering operation based on the wet system is executed only in the winter season. However, according to this embodiment, air filtering can be performed in the summer season without humidifying air, and air-conditioned and filtered air can be supplied to the room even in the summer season. Furthermore, since the operation of the air conditioner having the air filtering function according to the present invention can be executed even in the summer season, active oxygen species can be supplied to the gas-liquid contact member 56 of the air filtering unit 5 at all times, and breeding of various bacteria, fungus, etc. in the gas-liquid contact member 56 and the air filtering unit 5 can be prevented.

Furthermore, the indoor unit 2 of the air conditioner 100 according to this embodiment is equipped with the air filtering water supply pipe 51 for supplying air filtering water to the air filtering unit 5, and the air filtering water supply pipe 51 is connected to at least a pair of electrodes 71, 72 for electrolyzing tap water or the like to generate air filtering water containing hypochlorous acid or the like, and a flow rate adjusting valve 52 for adjusting the concentration of hypochlorous acid or the like in the air filtering water to a predetermined concentration by changing the flow rate of tape water or the like which passes between the electrodes 71, 72. Therefore, in accordance with the kind of the virus, etc., the air filtering water containing hypochlorous acid or the like whose concentration is set to such a concentration as to inactivate the virus, etc. can be generated. Accordingly, the air filtering water containing hypochlorous acid of the concentration concerned is supplied to the air filtering unit 5, and air is passed through the gas-liquid contact member 56 of the air filtering unit 5, whereby the target virus, etc. can be effectively inactivated. Furthermore, when odor components are passed through the gas-liquid contact member 56, the odor components react with hypochlorous acid or the like in the air filtering water, and the odor components are ionized and solved in the air filtering water, so that the odor components are removed from the air and thus the air is deodorized.

According to this embodiment, the filtering water containing hypochlorous acid or the like is discharged from the lower portion of the air filtering unit 5 to the drain pan 61. Therefore, the filtering water is contaminated into the drain water stocked in the drain pan 61, thereby preventing occurrence of various bacteria, fungus, etc. in the drain water and thus occurrence of slime on the drain pan 61. Therefore, the cleaning and maintenance frequency of the drain pan 61 is reduced, and thus the labor of the cleaning and maintenance work can be reduced.

Furthermore, according to this embodiment, the air filtering unit 5 is provided to the air blow-out port 22 side of the indoor unit 2, and thus hypochlorous acid, etc. contained in the air blown out from the air filtering unit 5 is not directly introduced into the indoor heat exchanger 24. Therefore, the indoor heat exchanger 24 can be prevented from being eroded by hypochlorous acid or the like.

According to this embodiment, the opening degree of the flow rate adjusting valve 52 is adjusted in accordance with the electrical conductivity of tap water or the like so that the concentration of hypochlorous acid or the like in the filtering air is adjusted to a predetermined concentration. For example, when virus as an inactivation target is specified and variation of the electrical conductivity of tap water or the like is little, the electrical conductivity may be measured when the air conditioner is set up, and the valve opening degree may be set to the valve opening degree corresponding to the measured electrical conductivity and the target concentration of hypochlorous acid or the like in advance. Furthermore, in this construction, the detection of the electrical conductivity of tap water or the like my be carried out at the time when the electrolysis of tap water or the like is started. However, it is unnecessary to detect the electrical conductivity every time the electrolysis is carried out because the electrical conductivity of tap water or the like is not greatly varied in a day, and the electrical conductivity of tap water or the like may be detected once per several times of electrolysis.

Furthermore, in this embodiment, the concentration of hypochlorous acid or the like in the air filteringwater is adjusted to the predetermined concentration by changing the flow rate of tap water or the like which is passed between the electrodes 71, 72. However, the concentration of hypochlorous acid or the like in the air f i ltering water may be adjusted to the predetermined concentration by changing the current flowing between the electrodes 71, 72 or the voltage applied between the electrodes 71, 72. According to this construction, even when the float rate adjusting valve 51 is not disposed in the filtering water supply pipe 51, the concentration of hypochlorous acid in the filtering water can be changed to a high concentration by increasing the current flowing between the electrodes 71, 72 (for example, 40mA/cm² in current derisity), for example. In this case, by merely using the existing electrodes 71, 72, the concentration of hypochlorous acid or the like in the filtering water can be freely changed. Therefore, the number of parts can be suppressed, and the cost and the space can be saved. Furthermore, this construction may be combined with the construction of the above embodiment described above. Accordingly to this construction, for example, filtering water containing a high concentration of hypochlorous acid can be generated.

Furthermore, according to this embodiment, for example, the concentration of hypochlorous acid in filtering water is adjusted to a predetermined concentration by changing the flow rate of tap water passing between the electrodes 71, 72. However, by changing the current supply time to the electrodes 71, 72, the concentration of hypochlorous acid in filtering water may be adjusted to a predetermined concentration, for example. According to this construction, the concentration of hypochlorous acid in filtering water may be changed with a simpler construction as compared with the construction that the current flowing between the electrodes 71, 72 or the voltage appliedbetween the electrodes is changed. By combining this construction with the construction of the above embodiment, the current time to the electrodes 71, 72 can be reduced, and the lifetime of the electrodes 71, 72 can be increased.

In the above embodiment, hypochlorous acid is generated as active oxygen species. However, Ozone (O₃) or hydrogen peroxide (H₂O₂) may be generated as active oxygen species. In this case, when platinum tantalum electrodes are used as the electrodes 71, 72, active oxygen species canbehighlyefficiently and stably generated from water in which ion species are rare.

At this time, at the anode, the following reaction occurs:

2H₂O → 4H⁺ + O₂ + 4e⁻

Simultaneously with the above reaction, the following reactions occur, and ozone (O₃) is generated.

3H₂O→ O₃ + 6H⁺ + 6e⁻

2H₂O→ O₃ + 4H⁺ + 4e⁻

Furthermore, at the cathode, the following reactions occur:

4H⁺ + 4e⁻ + (4OH⁻) → 2H₂ + (4OH⁻)

O₂⁻ + e⁻ + 2H⁺ → H₂O₂

That is, O₂⁻ generated through the electrode reaction and H⁺ in solution are bonded to each other to generate hydrogen peroxide (H₂O₂).

In this construction, ozone (O₃) and hydrogen peroxide (H₂O₂) which have strong sterilizing power are generated by supplying current to the electrodes 71, 72, and electrolytic water containing ozone (O₃) and hydrogen peroxide (H₂O₂) can be created. The concentration of ozone or hydrogen peroxide in the filtering water (electrolytic water) is adjusted to a value suitable for inactivate target virus or the like and air is passed through the gas-liquid contact member 56 supplied with the filtering water having this concentration, whereby target virus, etc. floating in the air can be inactivated. Furthermore, even odor reacts with ozone or hydrogen peroxide in the filtering water when passing through the gas-liquid contact member 56, and ionized and dissolved in the electrolytic water, whereby the odor components are removed from the air and thus the air is deodorized.

In this embodiment, the filtering water discharged from the air filtering unit 5 is stocked in the drain pan 61, and the discharged to the outside of the air conditioner through the drain pump 62 together with the drain water. However, a part or all of the drain water may be returned to the electrolytic unit 7, and reused. In this construction, the filtered water which has been used for air filtering is electrolyzed in the electrolysis unit 7 again, and thus various fungus, bacteria, etc. in the filtering water can be prevented from occurring in connection with the re-use. Furthermore, the supply flow amount of tap water or the like can be reduced by re-using the filtering water, and thus the energy can be saved.

When scale deposits on the electrode (cathode) by electrolyzing tap water, the electrical conductivity is lowered, and it is difficult to continue electrolysis. In this case, it is effective to invert the polarities of the electrodes 71, 72 (the plus and minus of the electrodes are switched to each other) . The scale depositing on the cathode can be removed by electrolyzing the cathode as the anode. With respect to this polarity inverting control, the inverting operation may be periodically carried out by using a timer, for example, or it may be carried out irregularly, for example, every time the operation is started. Furthermore, the increase of the electrolysis resistance (decrease of the electrolysis current, or increase of the electrolysis voltage) is detected, and the polarities may be inverted on the basis of the detection result.

Furthermore, in the above embodiment, one indoor unit 2 is connected to one outdoor unit 1. However, the present invention may be applied to a multi-type air conditioner in which plural indoorunits 2 are connected to one outdoor unit inparallel.

## Claims

1. An air conditioner comprising:
a refrigerant circuit including a compressor, an outdoor heat exchanger and an indoor heat exchanger which are successively connected to one another;
a housing for accommodating the indoor heat exchanger;
an air suction port for sucking air;
an air blow-out port for blowing out air to a room;
an air blowing fan for making air flow from the air suction port to the air blow-out port to form an air flow passage; and
an air filtering unit that is disposed in the air flowpassage extending from the air suction port to the airblow-out port and bringing air heat-exchanged by the indoor heat exchanger into contact with liquid having a filtering effect.

2. The air conditioner according to claim 1, wherein the air filtering unit is equipped with a gas-liquid contact member for bringing the air heat-exchanged by the indoor heat exchanger into contact with the liquid, anda liquid supplyunit for supplying the liquid to the gas-liquid contact member.

3. The air conditioner according to claim 1, wherein the liquid is electrolytic water containing active oxygen species.

4. The air conditioner according to claim 3, wherein the liquid supply unit comprises an electrolytic unit for electrolyzing tap water to generate the electrolytic water.

5. The air conditioner according to claim 4, wherein the electrolytic unit includes at least one pair of electrodes, the water being electrolyzed by supplying current to the electrodes.

6. The air conditioner according to claim 4, wherein the liquid supply unit further comprising an electrical conductivity meter for detecting the electrical conductivity of the tap water to be supplied to the electrolytic unit, and an flow rate adjusting valve for adjusting a flow rate of the tap water to be supplied to the electrolytic unit in accordance with the electrical conductivity detected by the electrical conductivity meter and a predetermined target concentration of the active oxygen species.

7. The air conditioner according to claim 3, wherein the active oxygen species contain at least one material selecting from the group consisting of hypochlorous acid, ozone and hydrogen peroxide.

8. The air conditioner according to claim 1, wherein the compressor and the outdoor heat exchanger is accommodated in an outdoor unit, and the housing, the indoor heat exchanger, the air blowing fan and the air filtering unit are accommodated in an indoor unit.

9. The air conditioner according to claim 1, wherein the air filtering unit is disposed at the downstream side of the indoor heat exchanger with respect to an air flow direction in the air flow passage.
